# EUROPEAN PATENT APPLICATION

(11) **EP 2 258 251 A1**
(43) Date of publication of application: **08.12.2010**
(21) Application number: 10164945.7
(22) Date of filing: 04.06.2010
(51) Int. Cl.: A61B 1/04

(54) **Image obtainment method and endoscopic apparatus**

(30) Priority: 05.06.2009 JP 2009135767
(71) Applicant: Fujifilm Corporation, Minato-ku Tokyo (JP)
(72) Inventor: Kubo, Masahiro, Ashigarakami-gun (JP); Okoyama, Kazuo, Ashigarakami-gun (JP); Ohta, Yasunori, Ashigarakami-gun (JP)
(74) Representative: Klunker . Schmitt-Nilson . Hirsch

(57) **Abstract**

An input of one of a plurality of combinations of wavelength components that have been set in advance is received. Further, a spectral estimation image signal obtainment mode and a narrow-band image signal obtainment mode are switched therebetween based on the combination of wavelength components that has been received to obtain a spectral estimation image signal or a narrow-band image signal.

## Description

### BACKGROUND OF THE INVENTION

### Field of the Invention

The present invention relates to an image obtainment method for obtaining image signals representing an image of an observation target, which are sequentially output from an imaging device. Further, the present invention relates to an endoscopic apparatus.

### Description of the Related Art

Conventionally, endoscopic apparatuses for observing tissues in the body cavities of patients were well known. Further, an electronic endoscopic apparatus that obtains an ordinary image by imaging an observation target in the body cavity by illuminating the observation target with white light and that displays the ordinary image of the observation target on a monitor screen is widely used.

For example, Japanese Unexamined Patent Publication No. 2002-095635 (Patent Document 1) proposes obtainment and display of a narrow-band image in which blood vessels in the superficial layer of a mucosa or the like are observable at high contrast. In Patent Document 1, the narrow-band image is obtained by illuminating a living body tissue with narrow-band light by using a narrow-band filter.

Further, Japanese Unexamined Patent Publication No. 2003-093336 (Patent Document 2) proposes obtainment and display of a spectral estimation image without using an optical narrow-band-pass filter. In Patent Document 2, the spectral estimation image similar to an image obtained by using a narrow-band-pass filter is obtained by performing matrix operation on color image signals obtained by imaging in a wide wavelength band.

However, an apparatus disclosed in Patent Document 1 obtains the narrow-band image by illuminating a subject with light that has passed through a narrow-band filter. Therefore, the amount of the narrow-band light illuminating the living body tissue is not sufficient, and the obtained image tends to be dark. The amount of light is especially small when a distant view image is obtained. Meanwhile, the spectral estimation image obtained by the apparatus disclosed in Patent Document 2 is sufficiently light. However, since wavelength estimation operation is performed by matrix operation, the accuracy of estimation is limited, and the image quality of the spectral estimation image is lower than that of a narrow-band image in some cases. The deterioration of the image quality is especially noticeable when a close-up / enlarged image is obtained.

### SUMMARY OF THE INVENTION

In view of the foregoing circumstances, it is an object of the present invention to provide an image obtainment method and an endoscopic apparatus that can obtain a more appropriate image based on an observation target.

An image obtainment method of the present invention is an image obtainment method in an endoscopic apparatus including a scope unit having an imaging device for obtaining an image of an observation target, wherein the observation target is illuminated with illumination light and narrow-band light, the wavelength band of which is narrower than that of the illumination light, in a switchable manner, and wherein a spectral estimation image signal obtainment mode, in which a spectral estimation image signal is obtained by illuminating the observation target with the illumination light and by performing spectral image processing on an image signal that is output from the imaging device by illumination with the illumination light by using a predetermined signal processing parameter, and a narrow-band image signal obtainment mode, in which the observation target is illuminated with the narrow-band light and a narrow-band image signal output from the imaging device by illumination with the narrow-band light is obtained, are switchable, the method comprising the steps of:
receiving an input of one of a plurality of combinations of wavelength components that have been set in advance; and
switching the spectral estimation image signal obtainment mode and the narrow-band image signal obtainment mode therebetween based on the received combination of wavelength components to obtain the spectral estimation signal or the narrow-band image signal.

An endoscopic apparatus of the present invention is an endoscopic apparatus comprising:
a light source unit that outputs illumination light and narrow-band light, the wavelength band of which is narrower than that of the illumination light, to an observation target in a switchable manner;
a scope unit including an imaging device that obtains an image of the observation target by receiving reflection light that has been reflected from the observation target by illumination of the observation target with the illumination light or the narrow-band light;
a spectral image obtainment unit that obtains a spectral estimation image signal by performing spectral image processing on an image signal output from the imaging device by illumination of the observation target with the illumination light by using a predetermined signal processing parameter;
a narrow-band image obtainment unit that obtains a narrow-band image signal output from the imaging device by illumination of the observation target with the narrow-band light;
a wavelength set receiving unit that receives an input of one of a plurality of combinations of wavelength components that have been set in advance; and
a control unit that switches, based on the combination of wavelength components received by the wavelength set receiving unit, the illumination light and the narrow-band light output from the light source unit, and that controls the light source unit, the spectral image obtainment unit, and the narrow-band image obtainment unit so that obtainment of the spectral estimation image signal and obtainment of the narrow-band image signal are switched.

In the endoscopic apparatus of the present invention, the scope unit may include a variable magnification optical system that forms, on the imaging device, an image of the observation target the magnification of which has been optically changed. Further, the control unit may change the magnification of the variable magnification optical system based on the combination of wavelength components received by the wavelength set receiving unit.

Further, a digital zoom processing unit that performs digital zoom processing on the image signal or the narrow-band image signal output from the imaging device may be provided, and the control unit may change the magnification of the digital zoom processing unit based on the combination of wavelength components received by the wavelength set receiving unit.

Further, a magnification adjustment unit that receives adjustment of the magnification after the magnification is changed may be provided.

Further, the plurality of combinations of wavelength components that have been set in advance may include a combination of wavelength components for enhancing a change in the tone of color (a hue or the like).

Alternatively, the plurality of combinations of wavelength components that have been set in advance may include a combination of wavelength components for enhancing a fine structure (a microstructure, a very small structure, or the like).

Alternatively, the plurality of combinations of wavelength components that have been set in advance may include a combination of wavelength components that can observe the distribution of a fine structure.

According to the image obtainment method and the endoscopic apparatus of the present invention, an input of one of a plurality of combinations of wavelength components that have been set in advance is received. Further, the spectral estimation image signal obtainment mode and the narrow-band image signal obtainment mode are switched therebetween based on the received combination of wavelength components to obtain the spectral estimation signal or the narrow-band image signal. Therefore, it is possible to obtain image signals appropriate for the combination of wavelength components and to display images that are more appropriate for image diagnosis (diagnosis using images).

For example, when the combination of wavelength components for enhancing a change in the tone of color (or hue) or the combination of wavelength components for enhancing a fine structure is input, it is possible to display, by obtaining spectral estimation image signals, images lighter than images represented by narrow-band image signals. Further, it is possible to more clearly recognize a change in the tone of color.

When a change in the tone of color needs to be observed, a distant view observation image may be obtained by setting the magnification of the variable magnification optical system approximately to one-power magnification (1x). Accordingly, an image that is more appropriate to observe the change in the tone of color is displayed. Further, when a fine structure, such as blood vessels in the superficial layer of a mucosa, is observed, a weakly magnified image (low magnification image) may be obtained by setting the magnification of the variable magnification optical system approximately to 40-powermagnification (40x) . Accordingly, an image that is appropriate to observe the fine structure is displayed.

When the combination of wavelength components that can observe the distribution of a fine structure is input, it is possible to more clearly observe a fine structure by obtaining narrow-band image signals instead of spectral estimation image signals. When the distribution of the fine structure is observed, a close-up / enlarged image may be obtained by setting the magnification of the variable magnification optical system approximately to 80-power magnification (80x). Accordingly, it is possible to display an image appropriate for observing the distribution of the fine structure.

### BRIEF DESCRIPTION OF THE DRAWINGS

Figure 1 is a schematic block diagram illustrating the configuration of an endoscopic system using an endoscopic apparatus according to an embodiment of the present invention;
Figure 2 is a diagram illustrating the structure of a rotary filter in the endoscopic system illustrated in Figure 1; and
Figure 3 is a diagram illustrating the optical characteristic of the rotary filter illustrated in Figure 2.

### DESCRIPTION OF THE PREFERRED EMBODIMENTS

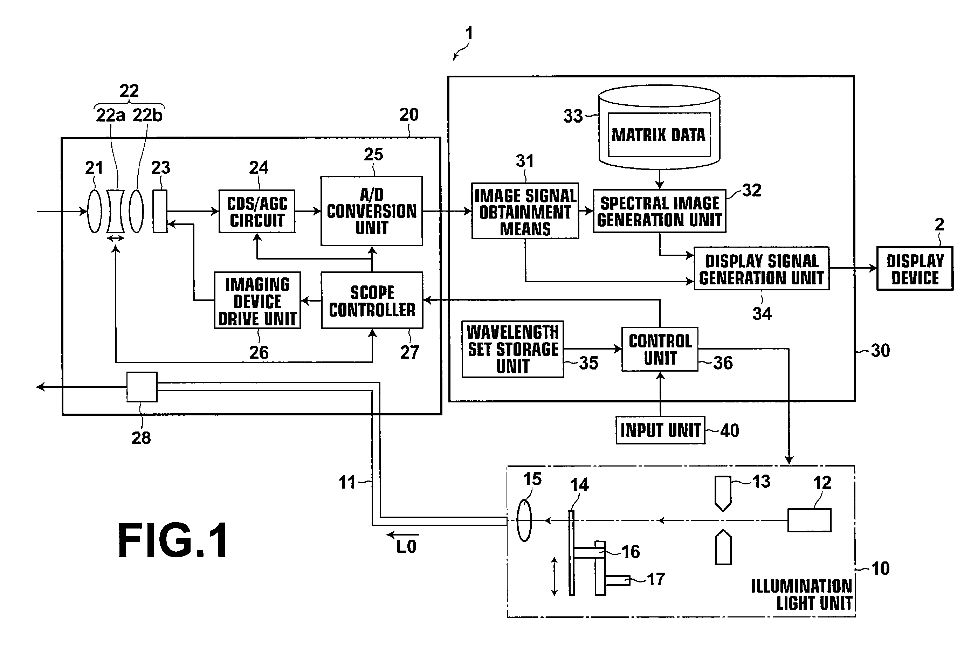

Hereinafter, an endoscopic system using an endoscopic apparatus according to an embodiment of the present invention will be described in detail with reference to drawings. Figure 1 is a schematic diagram illustrating an endoscopic system 1 using an endoscopic apparatus according to an embodiment of the present invention.

As illustrated in Figure 1, the endoscopic system 1 includes a scope unit 20, a processor unit 30, an illumination light unit 10, a display device 2, and an input unit 40. The scope unit 20 is inserted (introduced) into the body cavity of a patient to observe an observation target. The scope unit 20 is detachably connected to the processor unit 30. Further, the scope unit 20 is optically connected to the illumination light unit 10 in a detachable manner. The illumination light unit 10 outputs illumination light L0. The display device 2 displays an image of the observation target based on signals output from the processor unit 30. The input unit 40 receives an input of information, such as a wavelength set, which will be described later.

As illustrated in Figure 1, the illumination light unit 10 includes a xenon lamp 12, a diaphragm device 13, a rotary filter 14, a condensing lens 15, a rotary filter motor 16, and a filter movement motor 17. The xenon lamp 12 outputs white light, and the diaphragm device 13 controls the amount of white light that has been output from the xenon lamp 12. The rotary filter 14 changes the white light to frame sequential light. The condensing lens 15 condenses the frame sequential light generated through the rotary filter 14 onto an incident surface of a light guide 11 connected to the scope unit 20. The rotary filter motor 16 rotates the rotary filter 14, and the filter movement motor 17 moves the rotary filter 14 in the direction of the diameter of the rotary filter 14 (the direction indicated with an arrow in Figure 1, which is perpendicular to the optical path of the rotary filter 14).

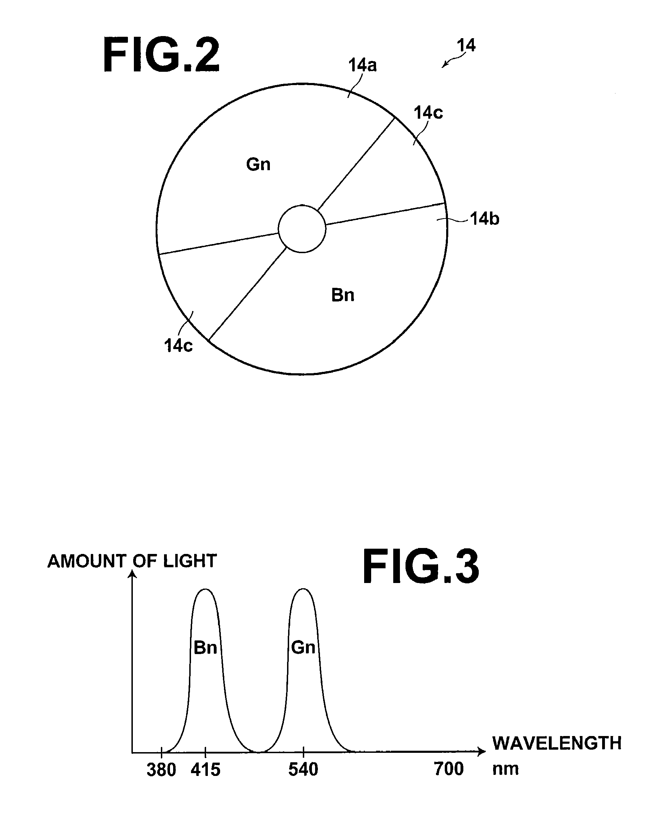

Figure 2 illustrates the structure of the rotary filter 14. As illustrated in Figure 2, the rotary filter 14 includes a Gn filter 14a, a Bn filter 14b, and light blocking portions 14c. The Gn filter 14a transmits light of a narrow-band wavelength component of green (hereinafter referred to as Gn component). The Bn filter 14b transmits light of a narrow-band wavelength component of blue (hereinafter referred to as Bn component). The light blocking portions 14c block light.

Figure 3 illustrates the spectral characteristic of narrow-band light output from each of the filters of the rotary filter 14. In Figure 3, Gn represents light transmitted through the Gn filter 14a, and Bn represents light transmitted through the Bn filter 14b.

Each of the filters of the rotary filter 14 is provided to obtain a narrow-band image. Specifically, for example, the Gn filter 14a may have a band-pass characteristic of passing light with a half width of 20 to 40 nm including the center wavelength of 540 nm, and the Bn filter 14b may have a band-pass characteristic of passing light with a half width of 20 to 40 nm including the center wavelength of 415 nm.

In the present embodiment, the filters are structured so as to have the aforementioned optical characteristics to obtain narrow-band images that can show a small structure, such as capillary vessels and a pit pattern. However, the kind of the narrow-band image is not limited to the aforementioned examples. A narrow-band filter having other color components or wavelength components may be used depending on the purpose of observation (use of the endoscopic apparatus).

The scope unit 20 includes an imaging optical system 21, a zoom lens 22, an imaging device 23, a CDS/AGC (correlated double sampling / automatic gain control) circuit 24, an A/D conversion unit 25, and an imaging device drive unit 26. Each of these elements is controlled by a scope controller 27.

The imaging device 23 is, for example, a CCD (charge coupled device), a CMOS (complementary metal oxide semiconductor), or the like. The imaging device 23 performs photoelectric conversion on an image of an observation target that has been formed by the imaging optical system 21 and the zoom lens 22, and outputs image signals representing the image of the observation target. As the imaging device 23, a primary-color-type imaging device including an RGB color filter may be used for example. Alternatively, a complementary-color-type imaging device may be used.

The operation of the imaging device 23 is controlled by the imaging device drive unit 26. The imaging device drive unit 26 outputs clock signals of a predetermined cycle to the imaging device 23, and the imaging device 23 sequentially outputs image signals based on the clock signals. For example, the cycle of the clock signals is set to 1/60 s based on the frame rate of 60 fps for displaying video images (motion images). Further, when the imaging device 23 outputs the image signals, the CDS/AGC circuit 24 performs sampling on the image signals, and amplifies the sampled image signals. The A/D conversion unit 25 performs A/D conversion on the image signals output from the CDS/AGC circuit 24, and outputs the image signals on which the A/D conversion has been performed to the processor unit 30.

The zoom lens 22 in the scope unit 20 changes the magnification based on a control signal output from the scope controller 27. Specifically, the zoom lens 22 includes a negative lens (a minus lens, or a concave lens) 22a and a positive lens (a plus lens, or a convex lens) 22b. The magnification is changed by moving the negative lens 22a in the direction of an arrow illustrated just under the negative lens 22a in Figure 1. In the present embodiment, the magnification of the zoom lens 22 is automatically changed based on the kind of the wavelength set input by an operator. Further, the magnification may be manually adjusted by the operator. The magnification is manually adjusted by an input of a magnification by the operator at the input unit 40.

Further, an illumination window 28 is provided at the leading end of the scope unit 20. The illumination window 28 faces an end of the light guide 11, the other end of which is connected to the illumination light unit 10.

The processor unit 30 includes an image signal obtainment unit 31, a spectral image generation unit 32, a storage unit 33, a display signal generation unit 34, a wavelength set storage unit 35, and a control unit 36. The image signal obtainment unit 31 obtains image signals output from the imaging device 23 of the scope unit 20 by illumination of the observation target with illumination light L0. The spectral image generation unit 32 performs spectral image processing on the image signals obtained by the image signal obtainment unit 31 by using estimation matrix data, thereby generating spectral estimation image signals of a predetermined wavelength component or components. The storage unit 33 stores the estimation matrix data for performing spectral image processing at the spectral image generation unit 32. The display signal generation unit 34 performs various kinds of processing on the image signals output from the image signal obtainment unit 31 or the spectral estimation image signals output from the spectral image generation unit 32 to generate image signals for display. The wavelength set storage unit 35 stores, in advance, a wavelength set table in which wavelength sets are correlated with imaging modes and the magnification of the zoom lens 22. The control unit 36 controls the processor unit 30, the scope unit 20, the illumination light unit 10, and the display device 2. The operation of these units will be described later in detail.

The display device 2 is a liquid crystal display device, a CRT (cathode-ray tube), or the like. The display device 2 can display, based on the image signals for display that have been output from the processor unit 30, ordinary images, spectral estimation images, narrow-band images, and the like.

Next, the operation of an endoscopic system according to the present embodiment will be described.

An endoscopic system 1 according to the present embodiment is configured in such a manner that an ordinary image display mode and a specific wavelength image display mode are switchable. The ordinary image display mode displays an ordinary image of the observation target, and the specific wavelength image display mode displays a specific wavelength image obtained by extracting a predetermined frequency component of the observation target. Further, in the specific wavelength image display mode, a narrow-band image imaging mode and a spectral estimation image imaging mode are switchable.

First, the action of the ordinary image display mode will be described.

First, the ordinary image display mode is selected by an operator at the input unit 40. After an insertion portion of the scope unit 20 is introduced into the body cavity of a patient, the xenon lamp 12 of the illumination light unit 10 is driven based on a control signal from the control unit 36 in the processor unit 30. The xenon lamp 12 outputs white light.

The rotary filter 14 has been moved, and is not located in the optical path of the white light output from the xenon lamp 12 at this time. Therefore, the white light output from the xenon lamp 12 does not pass through the rotary filter 14.

The white light output from the xenon lamp 12 enters an end of the light guide 11, and guided by the light guide 11. Further, the white light is output from the other end of the light guide 11, and illuminates the observation target through the illumination window 28. Further, reflection light L_{R} reflected from the observation target by illumination of the observation target with the white light enters the imaging optical system 21 and the zoom lens 22 in the scope unit 20. The imaging optical system 21 and the zoom lens 22 form an image of the observation target on an imaging plane of the imaging device 23. The magnification of the zoom lens 22 is set to 1× (one-power magnification) in the initial setting. After the ordinary image is displayed, the magnification may be changed to a desirable magnification by an input of the magnification by the operator at the input unit 40.

Further, the imaging device 23 that is driven by the imaging device drive unit 26 obtains an image of the observation target by imaging, and sequentially outputs image signals composed of R components, G components and B components based on clock signals output from the imaging device drive unit 26.

Correlated double sampling and amplification by automatic gain control are performed on the image signals by the CDS/AGC circuit 24. After then, A/D conversion is performed on the image signals by the A/D conversion unit 25 to convert the image signals into digital signals. The digital signals are output to the processor unit 30.

The image signal obtainment unit 31 in the processor unit 30 obtains the image signals composed of the R components, G components and B components, which have been output from the scope unit 20. The image signal obtainment unit 31 sequentially outputs the obtained image signals to the display signal generation unit 34.

The display signal generation unit 34 performs various kinds of signal processing on the input image signals, and generates Y/C signals composed of luminance signals Y and chrominance signals C. Further, various kinds of signal processing, such as I/P conversion and noise removal (reduction), are performed on the Y/C signals to generate image signals for display. The generated image signals for display are output to the display device 2.

The display device 2 displays an ordinary image of the observation target based on the input image signals for display.

Next, an action when the operator changes the ordinary image display mode to a specific wavelength image display mode while a video image of an ordinary image is displayed in the ordinary image display mode will be described.

First, the operator selects the specific wavelength image display mode at the input unit 40. Further, the operator selects and inputs one of a plurality of wavelength sets that have been set in advance. In the present embodiment, it is assumed that wavelength set 1 for enhancing a change in the tone of color (or hue), wavelength set 2 for enhancing a fine structure and wavelength set 3 for making it possible to observe the distribution of the fine structure are set in advance. Specifically, the wavelengths of 550 nm, 500 nm, and 470 nm are set as the wavelength set 1 for enhancing a change in the tone of color. The wavelengths of 525 nm, 495 nm, and 495 nm are set as the wavelength set 2 for enhancing the fine structure. The wavelengths of 540 nm, 415 nm, and 495 nm are set as the wavelength set 3 for making it possible to observe the distribution of the fine structure. In the present embodiment, the aforementioned three wavelength sets are set in advance. Alternatively, other wavelength sets may be set in advance based on the purpose of observation (use of the endoscopic apparatus).

When the operator inputs a wavelength set, the control unit 36 refers to the wavelength set table that is stored in the wavelength set storage unit 35 in advance, and obtains an imaging mode and the magnification of the zoom lens 22 corresponding to the input wavelength set. In the present embodiment, it is assumed that the wavelength set storage unit 35 stores, in advance, a wavelength table as shown in the following Table 1:

**[Table 1]**

| | Wavelength Set | Imaging Mode | Zoom Magnification |
|---|---|---|---|
| 1 | R(550 nm) | Spectral Estimation Image | 1x |
| | G(500 nm) | Imaging Mode | |
| | B(470 nm) | | |
| 2 | R(525 nm) | Spectral Estimation Image | 40x |
| | G(495 nm) | Imaging Mode | |
| | B(495 nm) | | |
| 3 | R(540 nm) | Narrow-Band Image Imaging Mode | 80x |
| | G(415 nm) | | |
| | B(415 nm) | | |

For example, when the operator selects and inputs the wavelength set 1, the control unit 36 obtains the spectral estimation image imaging mode as the imaging mode, and 1x (one-power magnification) as the zoom magnification (the magnification of the zoom lens) . When the operator selects and inputs the wavelength set 2, the control unit 36 obtains the spectral estimation image imaging mode as the imaging mode, and 40x (40-power, or 40 times) as the zoom magnification. When the operator selects and inputs the wavelength set 3, the control unit 36 obtains the narrow-band image imaging mode as the imaging mode, and 80x (80-power, or 80 times) as the zoom magnification.

Further, the control unit 36 controls each unit based on the imaging mode and the zoom magnification that have been obtained as described above.

First, an action when the wavelength set 1 is selected and input by the operator will be specifically described. When the wavelength set 1 is selected and input, first, the control unit 36 outputs a control signal to the scope controller 27 so that the magnification of the zoom lens 22 becomes 1x (one power magnification). The scope controller 27 controls the zoom lens 22 based on the input control signal. Further, the control unit 36 controls the system so that a spectral estimation image is obtained by imaging.

Specifically, the control unit 36 outputs a control signal to the image signal obtainment unit 31, and the image signal obtainment unit 31 outputs an image signal to the spectral image generation unit 32 based on the control signal.

Further, the spectral image generation unit 32 performs spectral image processing on the image signal composed of an R component, a G component, and a B component by using estimation matrix data stored in the storage unit 33.

Specifically, the spectral image generation unit 32 generates spectral estimation data (q1 through q59) by performing matrix operation represented by the following formula (1):

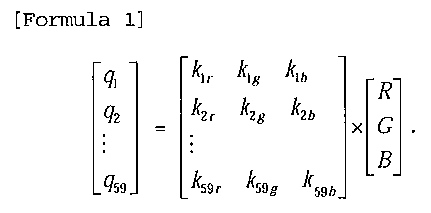

For example, the estimation matrix data includes 59 wavelength band parameters into which the wavelength band of 410 nm to 700 nm is divided at 5 nm intervals. Each of the wavelength band parameters includes coefficients kₚᵣ, k_{pg}, and k_{pb} (p = 1 to 59) .

Since the wavelength set 1 has been selected, the spectral image generation unit 32 obtains spectral estimation data q29, q19, and q13, which correspond to the wavelength bands of the wavelength set 1 (550 nm, 500 nm, 470 nm) respectively, from the spectral estimation data (q1 through q59).

Further, an appropriate gain and offset is applied to the calculated spectral estimation data q29, q19, and q13, and pseudo three color image signals R', G', and B' (spectral estimation image signals) are obtained respectively.

The pseudo three color image signals R', G', and B' are sequentially output to the display signal generation unit 34. The display signal generation unit 34 performs various kinds of signal processing on the input pseudo three color image signals R', G', and B', and generates a Y/C signal composed of luminance signal Y and chrominance signal C. Further, the display signal generation unit 34 performs various kinds of signal processing, such as IP conversion and nose removal (reduction), on the Y/C signal to generate an image signal for display. The generated image signal for display is output to the display device 2.

The display device 2 displays, based on the image signal for display that has been input, a spectral estimation image of a predetermined wavelength component or components of the observation target.

Next, an action when the wavelength set 2 is selected and input by the operator will be specifically described. When the wavelength set 2 is selected and input, first, the control unit 36 outputs a control signal to the scope controller 27 so that the magnification of the zoom lens 22 becomes 40x (40-power magnification) . The scope controller 27 controls the zoom lens 22 based on the input control signal. Further, the control unit 36 controls the system so that a spectral estimation image is obtained by imaging.

Specifically, the control unit 36 outputs a control signal to the image signal obtainment unit 31, and the image signal obtainment unit 31 outputs an image signal to the spectral image generation unit 32 based on the control signal.

Further, the spectral image generation unit 32 performs spectral image processing on the image signal composed of an R component, a G component, and a B component in a manner similar to the aforementioned method, and generates spectral estimation data (q1 through q59).

Further, the spectral image generation unit 32 obtains spectral estimation data q24, q18, and q18, which correspond to the respective wavelength bands of the wavelength set 2 (525 nm, 495 nm, 495 nm) respectively, from the spectral estimation data (q1 through q59).

Further, an appropriate gain and offset is applied to the calculated spectral estimation data q24, q18, and q18, and pseudo three color image signals R', G', and B' (spectral estimation image signals) are obtained respectively.

The pseudo three color image signals R', G', and B' are sequentially output to the display signal generation unit 34. The display signal generation unit 34 performs various kinds of signal processing on the input pseudo three color image signals R', G', and B', and generates a Y/C signal composed of luminance signal Y and chrominance signal C. Further, the display signal generation unit 34 performs various kinds of signal processing, such as IP conversion and nose removal (reduction), on the Y/C signal to generate an image signal for display. The generated image signal for display is output to the display device 2.

The display device 2 displays, based on the image signal for display that has been input, a spectral estimation image of a predetermined wavelength component or components of the observation target.

Next, an action when the wavelength set 3 is selected and input by the operator will be specifically described. When the wavelength set 3 is selected and input, first, the control unit 36 outputs a control signal to the scope controller 27 so that the magnification of the zoom lens 22 becomes 80x (80-power magnification) . The scope controller 27 controls the zoom lens 22 based on the input control signal. Further, the control unit 36 controls the system so that a narrow-band image is obtained by imaging.

Specifically, first, the control unit 36 outputs a control signal to the illumination light unit 10. The illumination light unit 10 moves, based on the input control signal, the rotary filter 14 into the optical path of white light, and makes the rotary filter 14 start rotating.

When the rotary filter 14 operates, Gn component light and Bn component light are sequentially output from the Gn filter 14a and the Bn filter 14b of the rotary filter 14, respectively. The output light enters an end of the light guide 11 through the condensing lens 15.

The Gn component light and the Bn component light that have been guided by the light guide 11 is output from the other end of the light guide 11, and illuminates the observation target through the illumination window 28. When the observation target is illuminated with the light, reflection light L_{G} and L_{B}, reflected from the observation target, sequentially enter the imaging optical system 21 of the scope unit 20. Further, the imaging optical system 21 and the zoom lens 22 form an image of the observation target on the imaging plane of the imaging device 23. Further, the imaging device 23 is driven by the imaging device drive unit 26, and obtains the image of the observation target by imaging. The imaging device 23 sequentially outputs image signals of the Gn component and Bn component. Further, the CDS/AGC circuit 24 performs correlated double sampling and amplification by automatic gain control on the image signals. After then, the A/D conversion unit 25 performs A/D conversion on the image signals, and inputs the image signals to the processor unit 30 as digital signals.

The image signals of the Gn component and the Bn component that have been output from the scope unit 20 are obtained by the image signal obtainment unit 31 in the processor unit 30. The image signal obtainment unit 31 outputs the image signals of the Gn component and the Bn component to the display signal generation unit 34.

The display signal generation unit 34 performs various kinds of signal processing on the respective image signals of the Gn component and the Bn component that have been input, and generates a Y/C signal composed of luminance signal Y and chrominance signal C. At this time, the image signal of the Gn component is used as an R signal, and the image signal of the Bn component is used as a G signal and a B signal. Further, various kinds of signal processing, such as I/P conversion and noise removal (reduction), is performed on the Y/C signals to generate image signals for display, and the image signals for display are output the display device 2. The display device 2 displays a narrow-band image of the observation target based on the image signals for display that have been input.

As described above, the magnification of the zoom lens 22 is controlled based on the wavelength set that has been selected and input by the operator. After the magnification is controlled to a magnification in the wavelength set table, the magnification may be further adjusted by an input of a magnification by the operator at the input unit 40.

In the above embodiments, the magnification of the zoom lens 22 is controlled based on the magnification corresponding to the wavelength set that has been selected and input by the operator. Instead of optically controlling the magnification in such a manner, the magnification may be controlled by digital zoom processing. Specifically, a digital zoom processing unit may be provided in the image signal obtainment unit 31, and the digital zoom processing unit may perform digital zoom processing based on a magnification corresponding to the wavelength set that has been selected and input by the operator.

## Claims

1. An image obtainment method in an endoscopic apparatus including a scope unit (20) having an imaging device (23) for obtaining an image of an observation target, wherein the observation target is illuminated with illumination light and narrow-band light, the wavelength band of which is narrower than that of the illumination light, in a switchable manner, and wherein a spectral estimation image signal obtainment mode, in which a spectral estimation image signal is obtained by illuminating the observation target with the illumination light and by performing spectral image processing on an image signal that is output from the imaging device (23) by illumination with the illumination light by using a predetermined signal processing parameter, and a narrow-band image signal obtainment mode, in which the observation target is illuminated with the narrow-band light and a narrow-band image signal output from the imaging device (23) by illumination with the narrow-band light is obtained, are switchable, the method comprising the steps of:
receiving an input of one of a plurality of combinations of wavelength components that have been set in advance; and
switching the spectral estimation image signal obtainment mode and the narrow-band image signal obtainment mode therebetween based on the received combination of wavelength components to obtain the spectral estimation signal or the narrow-band image signal.

2. An endoscopic apparatus comprising:
a light source unit (10) that outputs illumination light and narrow-band light, the wavelength band of which is narrower than that of the illumination light, to an observation target in a switchable manner;
a scope unit (20) including an imaging device (23) that obtains an image of the observation target by receiving reflection light that has been reflected from the observation target by illumination of the observation target with the illumination light or the narrow-band light;
a spectral image obtainment unit (32) that obtains a spectral estimation image signal by performing spectral image processing on an image signal output from the imaging device (23) by illumination of the observation target with the illumination light by using a predetermined signal processing parameter;
a narrow-band image obtainment unit (31) that obtains a narrow-band image signal output from the imaging device (23) by illumination of the observation target with the narrow-band light;
a wavelength set receiving unit (40) that receives an input of one of a plurality of combinations of wavelength components that have been set in advance; and
a control unit (36) that switches, based on the combination of wavelength components received by the wavelength set receiving unit (40), the illumination light and the narrow-band light output from the light source unit (10), and that controls the light source unit (10), the spectral image obtainment unit (32), and the narrow-band image obtainment unit (31) so that obtainment of the spectral estimation image signal and obtainment of the narrow-band image signal are switched.

3. An endoscopic apparatus, as defined in Claim 2, wherein the scope unit (20) includes a variable magnification optical system (22) that forms, on the imaging device (23), an image of the observation target the magnification of which has been optically changed, and wherein the control unit (36) changes the magnification of the variable magnification optical system (22) based on the combination of wavelength components received by the wavelength set receiving unit (40).

4. An endoscopic apparatus, as defined in Claim 2, further comprising:
a digital zoom processing unit (31) that performs digital zoom processing on the image signal or the narrow-band image signal output from the imaging device (23), wherein the control unit (36) changes the magnification of the digital zoom processing unit (31) based on the combination of wavelength components received by the wavelength set receiving unit (40).

5. An endoscopic apparatus, as defined in Claim 3 or 4, further comprising:
a magnification adjustment unit (40) that receives adjustment of the magnification after the magnification is changed.

6. An endoscopic apparatus, as defined in any one of Claims 2 to 5, wherein the plurality of combinations of wavelength components that have been set in advance includes a combination of wavelength components for enhancing a change in the tone of color.

7. An endoscopic apparatus, as defined in any one of Claims 2 to 6, wherein the plurality of combinations of wavelength components that have been set in advance includes a combination of wavelength components for enhancing a fine structure.

8. An endoscopic apparatus, as defined in any one of Claims 2 to 7, wherein the plurality of combinations of wavelength components that have been set in advance includes a combination of wavelength components that can observe the distribution of a fine structure.
